(19) Europäisches Patentamt
European Patent Office
Office européen des brevets

(11) Veröffentlichungsnummer: **0 144 884**
**B1**

(12) **EUROPÄISCHE PATENTSCHRIFT**

(45) Veröffentlichungstag der Patentschrift: **20.05.87**

(21) Anmeldenummer: **84114258.1**

(22) Anmeldetag: **26.11.84**

(51) Int. Cl.⁴: **C 07 C 102/00**, C 07 C 103/133, C 07 C 131/00

(54) **Verfahren zur Herstellung von alpha-substituierten Acrylsäureamiden.**

(30) Priorität: **02.12.83 DE 3343675**

(43) Veröffentlichungstag der Anmeldung: **19.06.85 Patentblatt 85/25**

(45) Bekanntmachung des Hinweises auf die Patenterteilung: **20.05.87 Patentblatt 87/21**

(84) Benannte Vertragsstaaten: **BE CH DE FR GB IT LI NL**

(56) Entgegenhaltungen:
**DE-A-1 044 795**
**DE-A-1 081 884**
**US-A-3 105 741**

(73) Patentinhaber: **BASF Aktiengesellschaft, Carl-Bosch- Strasse 38, D-6700 Ludwigshafen (DE)**

(72) Erfinder: **Merger, Franz, Dr.Chem., Max- Slevogt-Strasse 25, D-6710 Frankenthal (DE)**
Erfinder: **Schwarz, Wolfgang, Dr. Chem., Wesostrasse 132, D-7507 Pfinztal (DE)**

**EP 0 144 884 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft ein neues Verfahren zur Herstellung von α-substituierten Acrylsäureamiden, ausgehend von Oximen α-substituierter Acroleine.

α-substituierte Acrylamide können durch Amidierung der jeweiligen Ester mit Ammoniak oder durch Hydratisierung der entsprechenden Nitrile erhalten werden. Die Synthese der dazu benötigten Acrylester und Acrylnitrile ist aber nur begrenzt möglich und im wesentlichen auf die (Meth)acrylverbindungen beschränkt. Darüber hinaus ist die Amidierung technisch aufwendig und läuft nicht besonders selektiv ab, da Nebenreaktionen, wie Michael-Additionen auftreten können.

Nach dem Vorschlag der US-A-4 365 092 soll die Herstellung von Methacrylamid aus Methacrylsäuremethylester mit wäßriger Ammoniaklösung erfolgen. Hierbei sind jedoch lange Reaktionszeiten erforderlich und man benötigt relativ hohe Mengen teurer anionischer Tenside, welche die Abtrennung und Reinigung des Amids erschweren.

Ein anderer Syntheseweg für Methacrylamid verläuft ausgehend von Acetoncyanhydrin über das Zwischenprodukt Methacrylamid-sulfat. Bei dieser Verfahrensweise fallen jedoch große Mengen an Salzen an, zudem müssen aufwendige Reinigungsschritte durchgeführt werden.

Zur Herstellung von Säureamiden sind aus der Literatur auch Umlagerungen von Aldoximen bekannt (J.Am.Chem.Soc. 83, 1983 (1961); Rec.Trav.Chim. Pays Bas 95, 123 (1976) und 96, 142 (1977)). Die den Aldoximen zugrunde liegenden Aldehyde gehören zumeist der Klasse der gesättigten oder der aromatischen Aldehyde an. Als Katalysatoren dienen Salze von Nickel, Zink, Palladium, Kobalt oder Kupfer. Besonders wirksam erwiesen sich dabei Nickel(II)acetat und Palladium(II)acetat, während dagegen Kupfer(II)acetat bei Benzaldoxim nur Umsetzungen in unerwünschter Richtung oder mit schlechten Ausbeuten liefert.

In der JP-A-128 302/1977 wird neben der Umlagerung von gesättigten Aldoximen und Benzaldoximen auch die Umsetzung von Zimtaldehydoxim in Gegenwart von Kupferacetylacetonat beschrieben. Es hat sich aber gezeigt, daß die Anwendung dieses Komplexsalzes bei der Herstellung von α-alkylsubstituierten Acrylsäureamiden zu keinen befriedigenden Ergebnissen führt.

Schließlich wird in der DE-A-3 205 946 die Herstellung von Methacrylamid, ausgehend von Methacroleinoxim in Gegenwart eines Katalysators, auf Basis Kupfer/Chrom, vorgeschlagen. Für diesen Katalysator, der zweckmäßig in Form eines Trägerkatalysators eingesetzt werden soll, ist ein definiertes Molverhältnis von Kupfer zu Chrom erforderlich.

Bei diesem Verfahren liegt die Ausbeute an Methacrylamid aber lediglich bei 72 %. Zudem hat sich gezeigt, daß die Verwendung der dort genannten Kupfersalze, die den Kupferanteil des Katalysators ausmachen und die sich von den Carbonsäuren Ameisensäure, Essigsäure und Weinsäure ableiten, durchweg nicht die gewünschten optimalen Resultate liefert.

In Anbetracht der bisher bekannt gewordenen unbefriedigenden und meistens auf Methacrylamid beschränkten Synthesen vonα-alkylsubstituierten Acrylsäureamiden lag der Erfindung die Aufgabe zugrunde, solche Verbindungen auf einfachere und wirtschaftlichere Weise herzustellen.

Demgemäß wurde gefunden, daß manα-substituierte Acrylsäureamide der allgemeinen Formel 1

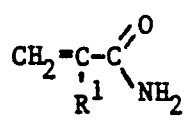 (I),

in der $R^1$ einen geradkettigen, verzweigten oder cyclischen Alkylrest mit bis zu 15 Kohlenstoffatomen bedeutet, der gegebenenfalls weiter substituiert sein kann, aus den Aldoximen der allgemeinen Formel II

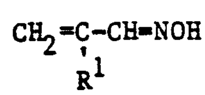 (II),

in der $R^1$ die obengenannte Bedeutung besitzt, vorteilhaft erhält, wenn man die Aldoxime in Gegenwart eines trägerfreien Kupfer(II)carboxylats einer Monocarbonsäure mit 5 bis 18 Kohlenstoffatomen auf eine Temperatur von 40 bis 250°C erwärmt.

Das erfindungsgemäße Verfahren wird mit Oximen von Acroleinen durchgeführt. die im α-Stellung einen geradkettigen, verzweigten oder cyclischen Alkylrest mit bis zu 15 Kohlenstoffatomen tragen. Dieser Alkylrest kann durch weitere Gruppen, die sich unter den Reaktionsbedingungen indifferent verhalten, substituiert sein, beispielsweise niedere Alkoxygruppen (auch in geminaler Stellung), niedere Acyloxygruppen, niedere Alkoxycarbonylgruppen oder niedere Mono- und Dialkylaminogruppen.

Beispielhaft seien folgendeα-alkylsubstituierten Acroleinoxime erwähnt: α Methacroleinoxim, α-

Ethylacroleinoxim, α-Butylacroleinoxim, α-(2-Ethylhexyl)acroleinoxim, α-Nonylacroleinoxim, α-Cyclohexylacroleinoxim, α-(4-Methylcyclohexyl)acroleinoxim, α-(3-Carbethoxypropyl)acroleinoxim oder α-(4,4-Dimethylaminobutyl)acroleinoxim.

Die den Aldoximen (II) zugrundeliegendenα-substituierten Acroleine sind mittels des in der EP-A-58 927 beschriebenen Verfahrens durch Umsetzung von Alkanalen mit Formaldehyd und sekumdärem Amim in Gegenwart von Säure gut zugänglich.

Die für das erfindungsgemäße Verfahren benötigten Aldoxime lassen sich nach an sich bekannten Methoden, wie sie beispielsweise in Houben-Weyl "Methoden der oganischen Chemie", Band 10/4, S. 55f. beschrieben sind, aus den α-substituierten Acroleinen und Hydroxylammoniumsalzen herstellen, wobei die freiwerdende Säure mit Base neutralisiert wird.

Es wurde weiter gefunden, daß man bei der Herstellung der niederen Homologen dieser Aldoxime, bei denen der gegebenenfalls substituierte α-Alkylrest bis zu 4 Kohlenstoffatome aufweist, überraschenderweise auf den üblichen Zusatz von Basen verzichten kann. Die Bildung und Abtrennung der Aldoxime erfolgt ohne Neutralisation der Säure und entsprechende Salzbildung. Die wäßrige Säure kann rückgeführt oder für Neutralisationszwecke verwendet werden.

Vorteilhaft lassen sich technische wäßrige Hydroxylammoniumsalzlösungen einsetzen. Überschüssige Säure und deren Ammoniumsalze, wie sie in solchen technischen Lösungen vorliegen, stören dabei nicht.

Die Umlagerung der Aldoxime zu den entsprechenden α-substituierten Acrylsäureamiden erfolgt bei einer Temperatur von 40 bis 250° C, vorzugsweise 60 bis 180° C und insbesondere 80 bis 150° C.

Vorzugsweise führt man die Umsetzung bei atmosphärischem Druck durch. In manchen Fällen ist es auch vorteilhaft sie unter erhöhtem Druck (bis zu 20 bar) vorzunehmen.

Man kann lösungsmittelfrei oder aber vorzugsweise in Gegenwart eines inerten Lösungsmittels wie Toluol, Xylol, Mesitylen, Chlorbenzol, Nitrobenzol, Tetralin, Decalin, Dioxan, Dibutylether oder Essigsäure-n-butylester arbeiten.

Erfindungsgemäß wird die Umlagerung der Aldoxime in Gegenwart eines trägerfreien Kupfer(II)carboxylats einer Monocarbonsäure mit 5 bis 18 Kohlenstoffatomen, vorzugsweise 6 bis 12 Kohlenstoffatomen und insbesondere 6 bis 10 Kohlenstoffatomen vorgenommen.

Als solche Säuren kommen (gegebenenfalls substituierte) Monocarbonsäuren mit einem gesättigten oder ungesättigten geradkettigen, verzweigten oder cyclischen Alkylrest, Aralkansäuren, Oxaalkansäuren sowie aromatische Carbonsäuren in Betracht, beispielsweise 2-Methylbutansäure, Pentansäure, Hexansäure, 2-Ethylhexansäure, 3,5,5-Trimethylhexansäure, Decansäure, 9-Decensäure, 9-Dodecensäure, 9-Octadecensäure, Cyclohexancarbonsäure, Phenylessigsäure, 1-Phenylcyclopentan-1-carbonsäure, Methoxyessigsäure, Benzoesäure, 3,5-Dichlorbenzoesäure oder Naphthoesäure.

Als besonders vorteilhaft erweist sich die Durchführung der Umsetzung in Gegenwart des Kupfer(II)salzes der 2-Ethylhexansäure oder der Decansäure.

Es zeigt sich, daß Kupfersalze von Monocarbonsäuren mit größerer Anzahl an Kohlenstoffatomen, im Vergleich zu den Carbonsäuresalzen, die eine geringe Zahl an Kohlenstoffatomen aufweisen, eine gesteigerte Wirkung besitzen (vgl. dazu die Zeichn.).

Aus den bislang bekannten Befunden bei der Umlagerung von gesättigten oder aromatischen Aldoximen konnte dieser, für das erfindungsgemäße Verfahren systemspezifische Effekt nicht entnommen werden, da z.B. bei der Umlagerung von Benzaldoxim die Verwendung von Nickel(II)formiat einerseits und Nickel(II)acetat andererseits völlig identische Ergebnisse lieferte (JA-A-128 302/1977).

Die Herstellung der für das erfindungsgemäße Verfahren benötigten Kupfersalze kann nach an sich bekannten Methoden, z.B. durch Umsetzung der Carbonsäuren mit Kupfer(II)carbonat oder durch Umsetzung von Kupfersalzen mit den Alkali- oder Ammoniumsalzen der jeweiligen Carbonsäure, erfolgen.

Die Kupfersalze werden trägerfrei eingesetzt, d.h. ohne Verwendung eines in der Katalysatortechmik häufig üblichen Trägermaterials und ihre Verwendung erfolgt zweckmäßig in Abwesenheit von Chrom.

Die Menge des eingesetzten Kupfer(II)carboxylats beträgt 0,5 bis 50 Mol%, bevorzugt 2 bis 10 Mol%, und insbesondere 3 bis 6 Mol%, jeweils bezogen auf 1 Mol Aldoxim.

Das erfindungsgemäße Verfahren wird üblicherweise so ausgeführt, daß man das Aldoxim zusammen mit Kupfer(II)carboxylat, vorzugsweise in einem inerten Lösungsmittel auf den genannten Temperaturbereich erwärmt. Dabei ist sowohl eine diskontinuierliche als auch eine kontinuierliche Arbeitsweise möglich. Zur besseren Kontrolle der exothermen Reaktion ist es zweckmäßig, nur einen Teil des Oxims vorzulegen und den Rest im Verlauf der Reaktion zuzugeben.

Nach Beendigung der Reaktion werden die Zielprodukte durch Kristallisation oder Extraktion abgetrennt und sind für viele Verwendungszwecke direkt einsetzbar. Sie können durch Umkristallisation gereinigt und gegebenenfalls durch Ionenaustausch von Kupferspuren befreit werden.

Besondere Vorteile des erfindungsgemäßen Verfahrens sind die Verwendung der im Vergleich zu den Acrylestern leicht zugänglichen Acroleine (die oft als Vorstufe für die Synthese der Acrylester dienen) sowie die unter den Reaktionsbedingungen besonders selektiv ablaufende Aldoximumlagerung.

Die nach dem erfindungsgemäßen Verfahren gewonnenen Amide sind wertvolle Zwischenprodukte für die Herstellung von modifizierbaren Polymeren, z.B. für Dispersionen und Lacke.

Die Erfindung soll durch folgende Beispiele veranschaulicht werden.

**Beispiel 1**

Herstellung von Methacroleinoxim

Zu 1510 ml einer Lösung, die 490,3 g Hydroxylammoniumsulfat, 19,25 g Schwefelsäure und 30,83 g Ammoniumsulfat enthielt, wurden innerhalb einer Stunde, bei ca. 3°C, unter kräftigem Rühren, 370 g Methacrolein (Reinheit 98%) zugetropft. Dann wurde die Kühlung entfernt und 2 Stunden weitergerührt, wobei die Temperatur des Reaktionsansatzes auf 21°C anstieg. Nach GC-Analyse waren 97% des Methacroleins umgesetzt. Man trennte die organische Phase ab, etherte die wäßrige Phase zweimal aus, trocknete die vereinigten organischen Phasen über Magnesiumsulfat und engte am Rotationsverdampfer ein. Der Rückstand wurde über einen Dünnschichtverdampfer (82°C/16 mbar) destilliert. Man erhielt 387 g Methacroleinoxim (Ausbeute 90%, bezogen auf umgesetztes Methacrolein; Reinheit 99,6%).

**Beispiel 2**

20 g Methacroleinoxim, 6 Mol% (bezogen auf Oxim) Cu(II)carboxylat (siehe Tabelle) und 13,35 g Diphenylether (interner Standard) wurden in 120 g o-Xylol unter Rühren auf 110°C erwärmt. Es setzte eine exotherme Reaktion ein, nach deren Abklingen man solange bei 110-C weiterreagieren ließ, bis alles Oxim verbraucht war (GC). Die angegebenen Ausbeuten beziehen sich auf quantitative GC-Analysen nach der Methode des internen Standards. Die Umsetzungen a) bis f) dienen als Vergleich.

**Tabelle**

| Cu(II)carboxylat | Reaktionsdauer [h] | Ausbeute Amid [%] |
|---|---|---|
| a) Cu-formiat | 10 | 41 |
| b) Cu-acetat | 5 | 62 |
| c) Cu-propionat | 1 | 67 |
| d) Cu-butyrat | 1 | 72 |
| e) Cu-tartrat | 4 | 0 |
| f) Cu-acetylacetonat | 4 | 58 |
| g) Cu-heptanoat | 1 | 79 |
| h) Cu-2-ethylhexanoat | 1 | 85 |
| j) Cu-decanoat | 1 | 89 |

**Beispiel 3**

Herstellung von Ethylacroleinoxim

Zu 2,7 1 einer Lösung, die 459,7 g Hydroxylammoniumsulfat, 18,1 g freie Schwefelsäure und 46,2 g Ammoniumsulfat enthielt, wurden bei einer Temperatur von 25°C innerhalb 8 min unter kräftigem Rühren, 414 g Ethylacrolein (Reinheit 98,5%) zugetropft. Anschließend wurde 2,5 Stunden bei 25°C nachgerührt. Man trennte die Phasen, schüttelte die wäßrige Phase mit Chloroform aus, trocknete die vereinigten organischen Phasen über Natriumsulfat und engte am Rotationsverdampfer ein. Nach Destillation über einen Dünnschichtverdampfer (105°C/32 mbar) erhielt man 436 g (95 %) Ethylacroleinoxim.

**Beispiel 4** (Vergleich)

23,3 g Ethylacroleinoxim und 6 Mol% (bezogen auf Oxim) Cu(II)-acetat wurden in 120 g Xylol auf 110°C erwärmt. Nach Abklingen der exothermen Reaktion wurde bei 110°C weitergerührt. Nach einer Stunde war die Umsetzung beendet. Man engte am Rotationsverdampfer ein und nahm den Rückstand in 250 ml Petrolether und 5 ml Methanol auf. Das beim Abkühlen auf -20°C ausgefallene rohe Ethylacrylamid wurde abgesaugt und getrocknet. Ausbeute: 16,5 g (70,8%)

**Beispiel 5**

Analog Beispiel 4; anstelle von 6 Mol% Cu(II)-acetat wurden 6 Mol% Cu(II)-2-ethylhexanoat eingesetzt. Ausbeute: 18,9 g (81,1%).

**Beispiel 6**

Zur siedenden Lösung von 49,4 g Cu(II)-2-ethylhexanoat (6 Mol.%, bezogen auf Oxim) in 1200 g o-Xylol wurden innerhalb von 15 min 403 g α-(3-Carbethoxypropyl)acroleinoxim zugegeben und nach beendeter Zugabe noch 10 min zum Rückfluß erhitzt. Danach kühlte man auf -20° C ab, mahm das ausgefallene rohe Amid in 1,5 l Wasser auf, gab die wäßrige Lösung auf einen sauren Kunstharzionenaustauscher und engte die erhaltene Lösung am Rotationsverdampfer ein. Man erhielt 317 g (77 %) einer schwach gelb gefärbten Flüssigkeit, die nach dem Animpfen vollständig durchkristallisierte, Fp. 48° C (aus Toluol).

**Patentansprüche**

1. Verfahren zur Herstellung von α-substituierten Acrylsäureamiden der allgemeinen Formel 1

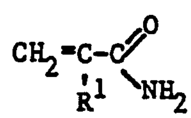

(I),

in der $R^1$ einen geradkettigen, verzweigten oder cyclischen Alkylrest mit bis zu 15 Kohlenstoffatomen bedeutet, der gegebenenfalls weiter substituiert sein kann, aus Aldoximen der allgemeinen Formel II

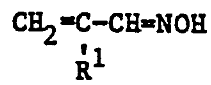

(II),

in der $R^1$ die obengenannte Bedeutung besitzt, dadurch gekennzeichnet, daß man die Aldoxime in Gegenwart eines trägerfreien Kupfer(II)carboxylats einer Monocarbonsäure mit 5 bis 18 Kohlenstoffatomen auf eine Temperatur von 40 bis 250° C erwärmt.

2. Verfahren gemäß Anspruch 1, dadurch gekennzeichnet, daß man die UUmsetzung bei einer Temperatur von 60 bis 180° C durchführt.

3. Verfahren gemäß Anspruch 1, dadurch gekennzeichnet, daß man die Umsetzung in Gegenwart eines Kupfer(II)carboxylats einer Monocarbonsäure mit 6 bis 12 Kohlenstoffatomen durchführt.

4. Verfahren gemäß Anspruch 1, dadurch gekennzeichnet, daß man die Umsetzung in Gegenwart eines Kupfer(II)carboxylats einer Monocarbonsäure mit 6 bis 10 Kohlenstoffatomen durchführt.

5. Verfahren gemäß Anspruch 1, dadurch gekennzeichnet, daß man die Umsetzung bei atmosphärischem Druck durchführt.

6. Verfahren gemäß Anspruch 1, dadurch gekennzeichnet, daß man die Umsetzung in Anwesenheit eines inerten Lösungsmittels durchführt.

**Claims**

1. A process for the preparation of an α-substituted acrylamide of the general formula I

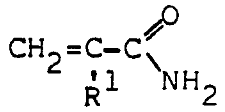

(I),

where $R^1$ is a straight-chain, branched or cyclic alkyl radical of up to 15 carbon atoms which can be unsubstituted or further substituted, from an aldoxime of the general formula II

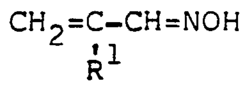

(II),

where $R^1$ has the above meaning, wherein the aldoxime is heated at from 40 to 250°C in the presence of a carrierfree copper(II) carboxylate obtained from a monocarboxylic acid of 5 to 18 carbon atoms.

2. A process as claimed in claim 1, wherein the reaction is carried out at from 60 to 180°C.

3. A process as claimed in claim 1, wherein the reaction is carried out in the presence of a copper(II) carboxylate obtained from a monocarboxylic acid of 6 to 12 carbon atoms.

4. A process as claimed in claim 1, wherein the reaction is carried out in the presence of a copper(II) carboxylate obtained from a monocarboxylic acid of from 6 to 10 carbon atoms.

5. A process as claimed in claim 1, wherein the reaction is carried out under atmospheric pressure.

6. A process as claimed in claim 1, wherein the reaction is carried out in the presence of an inert solvent.

**Revendications**

1. Procédé de préparation d'amides d'acides acryliques α-substitués de formule générale I

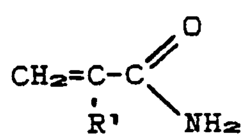

(I)

dans laquelle $R^1$ représente un radical alkyle à chaîne droite, ramifiée ou cyclique pouvant comporter jusqu'à 15 atomes de carbone et, le cas échéant, pouvant être encore substitué, à partir d'aldoximes de formule générale (II)

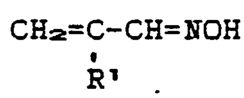

(II)

dans laquelle $R^1$ a la signification donnée ci-dessus, caractérisé en ce qu'on chauffe les aldoximes à une température de 40 à 250°C en présence d'un carboxylate cuivrique d'un acide monocarboxylique à 5 - 18 atomes de carbone, exempt de support.

2. Procédé selon la revendication 1, caractérisé en ce qu'on mène la réaction à une température de 60 à 180°C.

3. Procédé selon la revendication 1, caractérisé en ce qu'on mène la réaction en présence d'un carboxylate cuivrique d'un acide monocarboxylique à 6 - 12 atomes de carbone.

4. Procédé selon la revendication 1, caractérisé en ce qu'on mène la réaction en présence d'un carboxylate cuivrique d'un acide monocarboxylique à 6 - 10 atomes de carbone.

5. Procédé selon la revendication 1, caractérisé en ce qu'on mène la réaction à la pression atmosphérique.

6. Procédé selon la revendication 1, caractérisé en ce qu'on mène la réaction en présence d'un solvant inerte.